# EUROPÄISCHE PATENTSCHRIFT

(11) **EP 0 675 709 B1**
(45) Veröffentlichungstag und Bekanntmachung des Hinweises auf die Patenterteilung: **18.08.1999**
(21) Anmeldenummer: 94901750.3
(22) Anmeldetag: 10.12.1993
(51) Int. Cl.: A61K 9/107, A61K 9/14

(54) **LYOPILISIERTE, WIRKSTOFFHALTIGE EMULSION**
FREEZE-DRIED EMULSION CONTAINING AN ACTIVE SUBSTANCE
EMULSION LYOPHILISEE CONTENANT UNE SUBSTANCE ACTIVE

(30) Priorität: 24.12.1992 DE 4244122
(43) Veröffentlichungstag der Anmeldung: 11.10.1995
(73) Patentinhaber: SCHWARZ PHARMA AG, D-40789 Monheim/Rhld. (DE)
(72) Erfinder: SCHÜTZ, Andreas, D-50668 Köln (DE); MIKA, Hans-Jürgen, D-53229 Bonn (DE); SIEVERT, Frank, D-51399 Burscheid (DE); EMSCHERMANN, Bernhard, D-40597 Düsseldorf (DE)
(86) Internationale Anmeldenummer: DE9301188
(87) Internationale Veröffentlichungsnummer: WO9414418

(56) Entgegenhaltungen:
- EP-A- 0 159 237
- WO-A-92/17162
- DATABASE WPI Week 9313, Derwent Publications Ltd., London, GB; AN 93-104185 & JP,A,5 043 450 (GREEN CROSS CORP)

## Beschreibung

Die vorliegende Erfindung betrifft eine lyophilisierte Emulsion, die mit Wasser zur ursprünglichen Emulsion redispergiert werden kann.

Insbesondere betrifft die Erfindung eine einen Wirkstoff enthaltende Fettemulsion, deren äußere, wässrige Phase mittels Gefriertrocknung entfernt wurde und die durch Zugabe von Wasser spontan zur ursprünglichen Emulsion mit einer der Ausgangszubereitung entsprechenden Teilchengrößenverteilung redispergiert werden kann.

Emulsionen sind disperse Systeme, die aus zwei miteinander nicht mischbaren Flüssigkeiten bestehen, von denen eine die innere, dispergierte Phase, in der anderen der äußeren, geschlossenen Phase fein verteilt ist.

Fettemulsionen sind Emulsionssysteme, in denen die innere, dispergierte Phase aus sehr feinen Fettpartikeln besteht, die homogen in der aus Wasser bestehenden äußeren Phase verteilt vorliegen. Derartige Emulsionszubereitungen werden bevorzugt parenteral angewendet und kommen insbesondere zur intravenösen Ernährung von Patienten, die oral keine Nahrung aufnehmen können, zum Einsatz.

Intravenös applizierbare Fettemulsionen stellen hohe Anforderungen an die Verträglichkeit ihrer Inhaltsstoffe und die Teilchengröße der Fettpartikel. Als Fettkomponente kommen vorzugsweise Öle mit einem hohen Anteil an ungesättigten Fettsäuren wie Sojabohnen-, Safflor- und Baumwollöl, als Emulgatoren Lecithine wie Ei-, Soja- und Cerebrallecitin, ferner Antioxidantien wie Tocopherolacetat und weitere Hilfsstoffe zur Anwendung.

Die Fetteilchen sollten zur Vermeidung von Blutdruckveränderungen und Emboliegefahr eine mittlere Teilchengröße von 1 µm nicht überschreiten.

Die Emulsionsherstellung erfolgt üblicherweise durch Voremulgierung der erwärmten Öl- und Wasserphasen mit einem Mixer, gefolgt von Feinstemulgierung mit einem Hochdruckhomogenisator und anschließender Sterilisation mit überhitztem Wasserdampf.

Das "Handbook on Injectable Drugs" (American Society of Hospital Pharmacists, Seite 237-244 (1986), Lawrence A. Trissel) beschreibt einige kommerzielle verfügbare Zubereitungen. Sie enthalten Sojabohnenöl bzw. Saffloröl, Eilecithin, Glycerin und Wasser und haben mittlere Teilchengrößen von ≤ 0,5 µm.

Fettemulsionen wurden auch wiederholt als Trägersysteme für parenteral zu verabreichende lipophile Arzneistoffe eingesetzt. Ziel ist es dabei, die therapeutische Wirksamkeit und Sicherheit von Arzneistoffen durch kontrollierte Freigabe aus Emulsionssystemen zu erhöhen.

Entsprechend ihren Löslichkeitseigenschaften sind lipophile Wirkstoffe in Emulsionen teilweise oder vollständig in die Fettpartikel eingebettet. Damit wird ihr pharmakokinetisches Verhalten maßgeblich durch das pharmakokinetische Verhalten der Trägerzubereitungen, aus welcher der Wirkstoff erst freigesetzt wird, bestimmt. Durch verzögerte Freisetzung werden hohe lokale Wirkstoffkonzentrationen vermieden, der Abbau verringert und damit die Wirkungsdauer erhöht.

Besonders vorteilhaft sind solche Emulsionssysteme für Prostaglandine, insbesondere Prostaglandin E₁ (PGE₁). PGE₁ ist ein hochwirksames Gewebshormon, das erfolgreich z.B. zur Behandlung der arteriellen Verschlußkrankheit eingesetzt wird. Zur Anwendung kommt dabei ein PGE₁-α-Cyclodextrinkomplex, der in physiologischer Kochsalzlösung gelöst, parenteral, vorzugsweise intraarteriell, möglichst nahe der zu behandelnden Körperregion infundiert wird. Hohe Druckverhältnisse und geringe Verdünnungseffekte bei der intraarteriellen Infusion stellen jedoch hohe Anforderungen an die apparative Ausstattung und Qualifikation des behandelnden Arztes. Intravenöse Infusion ist zwar vergleichsweise einfacher durchzuführen, wegen lokal reizender Wirkung des PGE₁ kann jedoch auch hier nur langsam und in relativ hoher Verdünnung infundiert werden. Insgesamt führt die verlängerte Verweildauer des Wirkstoffes im Gefäßsystem vor Erreichen des Zielortes und insbesondere die zusätzliche Passage des Lungenkreislaufes zu erhöhtem Wirkstoffabbau. Sowohl intraarterielle als auch intravenöse Infusionen stellen hohe Anforderungen an Ausstattung und sorgfältige Einstellung der Infusionsrate und werden daher in der Regel stationär und nicht vom niedergelassenen Arzt durchgeführt, was einer breiten Anwendung des wertvollen Wirkstoffes in der Therapie der arteriellen Verschlußkrankheit entgegensteht.

Diese Probleme können durch Einbettung von PGE₁ in eine Fettemulsion umgangen werden. Durch verzögerte Wirkstofffreisetzung werden hohe lokale Konzentrationen vermieden, der Wirkstoffabbau verringert und die Wirkungsdauer erhöht, so daß solche Formulierungen auch zur intravenösen Bolusinjektion geeignet sind.

Der Herstellungsprozeß solcher wirkstoffhaltigen Fettemulsionen entspricht weitgehend der o.a. Herstellung einer Fettemulsion mit dem Unterschied, daß der einzubettende Wirkstoff vor Durchführung der Voremulgierung in der Ölphase gelöst wird. Derartige PGE₁ enthaltende Fettemulsionen sind zwar geeignet, die beschriebenen Nachteile der herkömmlichen Anwendung von PGE₁ zu lösen, weisen aber eine geringe Lagerstabilität infolge von hydrolytischem Abbau des Wirkstoffs auf, was ihrer allgemeinen Verwendbarkeit entgegensteht.

Eine Möglichkeit zur Stabilisierung des Wirkstoffs innerhalb der Emulsion liegt im Entfernen von den Wirkstoff destabilisierenden Substanzen. Ein Beispiel hierfür gibt US-P 4,684,633, die beschreibt, daß Verwendung von phosphatidylethanolaminfreiem Eilecithin in einer Prostaglandin, Sojaöl, Eilecithin, Glycerin und Wasser enthaltenden Emulsionszusammensetzung Wirkstoffstabilisierung bewirkt. Wirkstoffstabilisierung wird jedoch nur für die Bedingung einer Kurzzeitsterilisation über 2,2 min bei 125°C gezeigt. Daten zur Stabilität bei Langzeitlagerung fehlen. Die Formulierung enthält auch Wasser, so daß Wirkstoffabbau infolge Hydrolyse grundsätzlich nicht ausgeschlossen werden kann. Ein grundlegender Nachteil ist, daß die Stabilisierung zunächst nur für die genannten Wirkstoffe gültig und nicht generell übertragbar ist.

Neben Stabilisierung des Wirkstoffes in einer applikationsfertigen Fettemulsion können Fettemulsionen mit intaktem Wirkstoff auch dadurch zur Anwendung gebracht werden, daß diese erst unmittelbar vor Gebrauch hergestellt werden. Ein Beispiel hierfür gibt die EP 0 331 755. Sie beschreibt einen Kit, bestehend aus einer üblichen Fettemulsion und entweder einer Wirkstofflösung in Wasser, flüssigen Polyalkylenglykolen,flüssigen Alkylethanolaminen oder flüssigen mehrere Hydroxylgruppen enthaltenden Alkoholen oder einer Wirkstoffzusammensetzung, bestehend aus Wirkstoff, Sacchariden und/oder Aminosäuren, die unmittelbar vor Anwendung vereinigt und intensiv durchgemischt werden. Intensive Durchmischung ist unbedingt erforderlich, um die Wirkstoffverteilung in die Fettemulsion zu ermöglichen. So erfolgt die Herstellung der in Ausführungsbeispiel 5 beschriebenen Wirkstoffemulsion beispielsweise durch Mischen über 2-3 Minuten. Lange Durchmischungszeiten sind bei der Anwendung jedoch von Nachteil.

Ein in EP 0 331 755, Beispiel 3, genanntes, Prostaglandine betreffendes Ausführungsbeispiel beschreibt eine Wirkstoffzusammensetzung, bestehend aus einem Prostaglandin und Triethanolamin. Triethanolamin ist jedoch physiologisch nicht unbedenklich, so daß seine Verwendung in pharmazeutischen Zubereitungen und insbesondere bei Injektabilia möglichst zu vermeiden ist.

Es bestand daher weiterhin Bedarf nach einer Emulsionsformulierung, die PGE₁-Abbau infolge Hydrolyse vermeidet, physiologisch unbedenklich und einfach zu handhaben ist. Eine solche Formulierung sollte hydrolytischen Wirkstoffabbau bei Lagerung durch Wasserfreiheit grundsätzlich ausschließen, durch Wasserzusatz leicht redispergierbar sein und die Verteilung des Wirkstoffes in die Fettphase der Emulsion von Anfang an sicherstellen.

Die Herstellung einer solchen Formulierung soll durch Lyophilisation einer Wirkstoff enthaltenden Fettemulsion erfolgen, die als wasserfreie Formulierung Lagerstabilität erwarten läßt und vor Applikation mit Wasser zur ursprünglichen Formulierung rekonstituiert werden kann. Untersuchungen zeigen jedoch, daß Lyophilisation von Emulsionen zu einem Ineinanderfließen der Fettpartikelchen und dadurch zu Vergrößerung von deren Teilchen oder gar zu deren völligen Zerstörung führt. Durch Zusatz von Gefrierschutzmitteln haben verschiedene Arbeitsgruppen wiederholt versucht, dieses Ineinanderfließen von Emulsionen infolge Lyophilisation zu verhindern. Ihre Ergebnisse zeigen jedoch, daß auch diese Maßnahme eine Zunahme des mittleren Teilchendurchmessers nicht verhindern kann [Lladser, M. et al.; The use of supports in the lyophilisation of oil-in-water emulsions, J. Pharm. Pharmacol 20, 450-455 (1968); Rambhan, D. et al.; Stability Studies on Lyophilized O/W Emulsions, Indian J. Pharm. 39, 52-55 (1977)]. Bensouda et al. zeigen weiterhin, daß die Zunahme der mittleren Teilchendurchmesser infolge Lyophilisation mit abnehmender Größe der Emulsionsteilchen zunimmt. [Bensouda, Y. et al.: Freeze-drying of Emulsing-Influence of Congealing on Granulometry Research of a Cryoprotection Agent, Pharm. Acta Helv. 64, 40-44 (1989)]. Emulsionen zur intravenösen Applikation sollten jedoch zur Vermeidung einer Embolie eine mittlere Teilchengröße von kleiner gleich 1 µm aufweisen. Verhinderung einer Teilchenvergrößerung infolge Lyophilisation erscheint daher besonders schwierig, ist aber aus Gründen der Sicherheit unverzichtbar.

Lyophilisierte rekonstituierbare Emulsionen, die auch zur intravenösen Anwendung geeignet sein sollen, beschreiben die Dokumente JP 60239417 und ZA 86 04 032. JP 60239417 beschreibt die Herstellung lyophilisierter Emulsionssysteme unter Zusatz von Gefrierschutzmitteln wie Saccharide und Zuckeralkohole und wasserlöslichen Polymeren wie Polyvinylpyrrolidon, Gelatine und Hydroxypropylcellulose. Den Ausführungsbeispielen läßt sich jedoch entnehmen, daß die Partikeldurchmesser der redispergierten Emulsionen im Vergleich zur Ausgangsemulsion durchweg höher liegen. ZA 86 04 032 offenbart lyophilisierte Emulsionszusammensetzungen und eine Methode zu ihrer Herstellung. Nach Herstellung einer üblichen Fettemulsion gemaß den bekannten Verfahren und Zusatz eines gerüstaufbauenden Zuckers wird die Emulsion in eine siedende Flüssigkeit mit einem Siedepunkt unterhalb -20°C gesprüht und anschließend lyophilisiert. Die Ausführungsbeispiele zeigen jedoch, daß auch in diesem Falle der mittlere Teilchendurchmesser der Fettpartikel nach Rekonstitution mit Wasser im Vergleich zur Ausgangsemulsion erhöht ist.

WO 92/17162 A1 beschreibt die Lyophilisation herkömmlicher Fettemulsionen mit definierten Herstellungsparametern. EP 0 159 237 A1 beschreibt die Gefriertrocknung von Öl-in-Wasser Emulsionen, die für die orale Anwendung bestimmt sind.

Lyophilisierte Emulsionen, die nach Rekonstitution mit Wasser eine der Ausgangszubereitung entsprechende Teilchenverteilung aufweisen, sind im Stand der Technik nicht enthalten.

Überraschenderweise wurde nun eine wirkstoffhaltige, wasserfreie, lyophilisierte Emulsionszusammensetzung gefunden, die durch Zugabe von Wasser zu der ursprünglichen, Wasser enthaltenden, wirkstoffhaltigen Emulsion mit entsprechender Teilchengrößenverteilung redispergierbar ist. Diese wirkstoffhaltige, wasserfreie, lyophilisierte Emulsionszusammensetzung besteht aus mindestens einem Wirkstoff, mindestens einem Gefrierschutzmittel und/oder mindestens einem Gerüstbildner, Glycerinpolyethylenglykolrizinoleat oder Polyoxyethylen-660-12-Hydroxystearat sowie diacetylierten Monoglyceriden oder einem Gemisch aus diacetylierten und partialacetylierten Monoglyceriden, wobei Glycerinpolyethylenglykolrizinoleat bzw. Polyoxyethylen-660-12-Hydroxystearat und diacetylierte Monoglyceride bzw. das Gemisch aus diacetylierten und partialacetylierten Monoglyceriden zueinander in einem Gewichtsverhältnis von 2:3 vorliegen.

Als wasserfrei wird eine Emulsionszusammensetzung bezeichnet, die keine zusammenhängende äußere wässrige Phase aufweist. Eine solche Zusammensetzung vermeidet hydrolytischen Abbau des/der Wirkstoffe/s und weist hohe Lagerstabilität auf.

Nach einer vorteilhaften Ausführungsform enthält das Gemisch aus diacetylierten und partialacetylierten Monoglyceriden 2 Gew.-% bis 40 Gew.-%, vorzugsweise 20 Gew.-% partialacetylierte Monoglyceride.

Nach einer besonders bevorzugten Ausführungsform der erfindungsgemäßen wasserfreien Emulsionszusammensetzung enthält diese mindestens einen Wirkstoff aus der Wirkstoffgruppe der Prostaglandine, insbesondere PGE₁.

Eine weitere vorteilhafte Ausführungsform der erfindungsgemäßen Emulsionszusammensetzung enthält als Gefrierschutzmittel/Gerüstbildner physiologisch verträgliche Mono-, Di- oder Oligosaccharide, insbesondere Laktose oder Zuckeralkohole wie Sorbit und/oder Mannit.

Nach einer weiteren bevorzugten Ausführungsform der erfindungsgemäßen Emulsionszusammensetzung enthält diese mindestens ein übliches Antioxidans, vorteilhafterweise aus der Gruppe der Tocopherole, wie α-, β-, γ- oder δ-Tocopherol, vorzugsweise α-Tocopherol sowie deren physiologisch verträgliche Salze wie Phosphate, Succinate und Acetate und/oder physiologisch verträgliche Puffersalze.

Nach einer weiteren bevorzugten Ausführungsform der erfindungsgemäßen Emulsionszusammensetzung weist deren innere, disperse Phase nach Rekonstitution mit Wasser mittlere Teilchendurchmesser von 0,1 µm bis 5 µm, vorzugsweise 0,2 µm bis 1,0 µm auf.

Die erfindungsgemäße Emulsionszusammensetzung kann hergestellt werden, indem einer mittels der in der Arzneimittelherstellung gebräuchlichen Verfahren und Technologien hergestellten Emulsion die wässrige Phase durch Lyophilisation entfernt wird.

Gegenstand der Erfindung ist daher auch ein Verfahren zur Herstellung der erfindungsgemäßen wirkstoffhaltigen, wasserfreien Emulsionszusammensetzung, welches dadurch gekennzeichnet ist, daß in üblicher Weise eine wirkstoffhaltige Emulsion hergestellt wird, deren äußere, wässrige Phase anschließend mittels Gefriertrocknung entfernt wird.

Durch Lösen in der wässrigen Phase können hydrophile Wirkstoffe, durch Lösen in der Emulgator und Fett enthaltenden Phase lipophile Wirkstoffe in die erfindungsgemäße Emulsionszusammensetzung eingearbeitet werden. Alternativ kann die Wirkstoffzugabe auch unmittelbar vor Durchführung der Lyophilisation erfolgen, was insbesondere für hydrolyseempfindliche und/oder thermolabile Wirkstoffe vorteilhaft ist. Damit kann das Verfahren zur Herstellung der erfindungsgemäßen wirkstoffhaltigen Emulsionszusammensetzung in vorteilhafter Weise an die physikochemischen Eigenschaften der Wirkstoffe angepaßt werden. Daher wird in einer zweckmäßigen Ausführungsform des erfindungsgemäßen Verfahrens mindestens ein Wirkstoff vor Emulgierung entweder in der wässrigen Phase oder in der Emulgator und Fett enthaltenden Phase gelöst oder mindestens ein Wirkstoff der Emulsion vor ihrer Gefriertrocknung zugegeben. Hierbei ist zu beachten, daß die Wirkstoffverteilung innerhalb des dispersen Systems vor Lyophilisation gewährleistet ist, was mit üblichen Methoden wie Gleichgewichtsdialyse, Differentialdialyse und Ultrafiltration leicht geprüft werden kann.

### Herstellung

### Ausführungsbeispiel 1 - Emulsionsherstellung

3,42 g Citronensäure-monohydrat, 1,57 g Tri-Natrium-citratdihydrat und 60,0 g Laktose wurden unter Erwärmen in 475 g Wasser für Injektionszwecke gelöst. Anschließend wurden 12,0 g Polyoxyethylen-660-12-Hydroxystearat und 18,0 g diacetylierte Monoglyceride mit einer Hydroxylzahl von 25 unter Erwärmen auf ungefähr 30°C und Inertatmosphäre (Stickstoff) in 30,0 g absolutem Ethanol gelöst.

Die Wasserphase wurde in ein geeignetes vorsterilisiertes Reaktionsgefäß (IKA-Laborreaktor LR-A 1000, IKA-Werke, Jahnke & Kunkel GmbH, Staufen, Deutschland) mit Temperiereinrichtung, Rührerwerkzeug und Zahnkranzdispergierer (Ultraturrax, IKA-Werke, Jahnke & Kunkel GmbH, Staufen, Deutschland) überführt und unter Rühren bei einem Vakuum von < 1 mbar auf 80°C erhitzt. Unter Aufrechterhaltung des Vakuums und Rührens wurde die ethanolische Emulgator-Lipidphase langsam über eine Kanüle direkt in die Wasserphase injiziert, wobei gleichzeitig intensiv mittels Ultraturrax homogenisiert wurde. Anschließend wurde unter ständigem Rühren und Fortbestand des Vakuums auf Raumtemperatur abgekühlt, währenddessen in mehreren Intervallen ca. 1 Min. lang mittels Zahnkranzdispergierstab intensiv homogenisiert wurde. Die abgekühlte Emulsion wurde in eine vorsterilisierte Flasche überführt und bis zur Weiterverarbeitung im Kühlschrank gelagert.

Zur Wirkstoffeinarbeitung wurden 163,9 mg PGE₁-α-Cyclodextrin gelöst in 5 ml Wasser für Injektionszwecke in einem vorsterilisierten 500 ml Meßkolben vorgelegt und mit der hergestellten Emulsion ad 500 ml aufgefüllt und eine Probe für Partikelmessungen entnommen. Die wirkstoffhaltige Lösung wurde unter aseptischen Bedingungen und Benutzung einer Dispensette mit einem Füllvolumen von 2 ml/Einzeldosis ca. 1 cm hoch in vorsterilisierte Vials überführt. Hierauf wurden die befüllten Vials mit Stopfen versehen, in den Lyophilisator gestellt und über 5 Stunden bei -45°C eingefroren. Der anschließend durchgeführte Lyophilisationsprozess erfolgte gemäß nachfolgender Tabelle.

| Zeit (Stunden) | Start-temperatur (°C) | End-temperatur (°C) | Druck (µbar) |
|---|---|---|---|
| 30 | - 40 | - 40 | 100 |
| 10 | - 40 | 25 | 100 |
| 15 | 25 | 25 | 1 |

Anschließend wurde das Vakuum unter gleichzeitigem Einleiten von Stickstoff entfernt, die Vials durch hydraulische Absenkung der Stopfen verschlossen und dem Lyophilisator nach dessen Öffnung unter keimarmen Bedingungen entnommen.

Sie enthielten trocken und gleichmäßig aussehende Kuchen, die durch Zugabe von Wasser spontan zerfielen und eine Emulsion ausbildeten.

### Ausführungsbeispiel 2

1,5 g Citronensäure-monohydrat, 2,34 g Tri-Natrium-citrat-dihydrat und 60 g Laktose wurden unter Erwärmen in 476 g Wasser für Injektionszwecke gelöst. Weiterhin wurden 12,0 g Glycerinpolyethylenglykolrizinoleat und 18,0 g diaycetylierte Monoglyceride mit einer Hydroxylzahl von 4 unter leichtem Erwärmen und Stickstoffatmosphäre in 30 g absolutem Ethanol gelöst. Anschließend wurde entsprechend dem in Ausführungsbeispiel 1 beschriebenen Herstellverfahren die Emulsion hergestellt, der Wirkstoff eingearbeitet, Proben zur Partikelmessung entnommen und lyophilisiert.

Ebenso wie in Ausführungsbeispiel 1 wurden trocken und gleichmäßig aussehende Produkt-Kuchen gebildet, die durch Zugabe von Wasser spontan zu einer Emulsion zerfielen.

### Teilchengrößenverteilung

Zur Prüfung des Einflusses von Lyophilisation und Redispersion auf die Teilchengröße wurden die Volumenverteilungen der Emulsionsteilchen vor Lyophilisation und nach erfolgter Gefriertrocknung und Redispersion mit Wasser mittels Laserlichtstreuung bestimmt (Malvern Master Sizer, Serie 3.01, Malvern Instruments Limited, Spring Lane South, Malvern, Worcestershire, WR14 1AQ, UK). Der Vergleich der Volumenverteilungen offenbart bereits geringste Veränderungen der besonders kritischen größeren Teilchen, da diese naturgemäß einen größeren Teil zum Gesamtvolumen beitragen. In nachfolgender Tabelle werden die Meßergebnisse der Teilchengrößenbestimmung gemäß den Ausführungsbeispielen erfindungsgemäß hergestellter Emulsionen dargestellt. Sie beinhalten die die Volumenverteilungen charakterisierenden maximalen Teilchengrößen, die zusammen mit den jeweilig darunter liegenden kleineren Teilchen 10 %, 50 %, 90 % oder 99 % des Gesamtvolumens umfassen.

| | maximale Größe der Teilchen, die zusammen mit den jeweils kleineren Teilchen folgende Volumenanteile umfassen | | | |
|---|---|---|---|---|
| | 10 % | 50 % | 90 % | 99 % |
| | | | | |

| **Ausführungsbeispiel 1** | | | | |
|---|---|---|---|---|
| vor Lyophilisation | 0,20 µm | 0,39 µm | 0,98 µm | 5,07 µm |
| nach Lyophilisation und Redispergierung mit Wasser | 0,19 µm | 0,35 µm | 1,02 µm | 3,32 µm |

| **Ausführungsbeispiel 2** | | | | |
|---|---|---|---|---|
| vor Lyophilisation | 0,19 µm | 0,38 µm | 1,09 µm | 4,56 µm |
| nach Lyophilisation und Redispergierung mit Wasser | 0,20 µm | 0,40 µm | 1,17 µm | 4,56 µm |

Es wird deutlich, daß die Teilchengrößenverteilungen der durch Zugabe von Wasser rekonstituierten Emulsionen mit den Teilchengrößenverteilungen der Ausgangsemulsionen übereinstimmen.

## Patentansprüche

1. Wirkstoffhaltige, wasserfreie, lyophilisierte Emulsionszusammensetzung, dadurch gekennzeichnet, daß diese aus mindestens einem Wirkstoff, mindestens einem Gefrierschutzmittel und/oder mindestens einem Gerüstbildner, Glycerinpolyethylenglykolrizinoleat oder Polyoxyethylen-660-12-Hydroxystearat sowie diacetylierten Monoglyceriden oder einem Gemisch aus diacetylierten und partialacetylierten Monoglyceriden besteht, wobei Glycerinpolyethylenglykolrizinoleat bzw. Polyoxyethylen-660-12-Hydroxystearat und diacetylierte Monoglyceride bzw. das Gemisch aus diacetylierten und partialacetylierten Monoglyceriden zueinander in einem Gewichtsverhältnis von 2:3 vorliegen, und nach Rekonstitution mit Wasser eine der Ausgangsemulsion entsprechende Teilchengrößenverteilung aufweist.

2. Wirkstoffhaltige, wasserfreie, lyophilisierte Emulsionszusammensetzung nach Anspruch 1, dadurch gekennzeichnet, daß das Gemisch aus diacetylierten und partialacetylierten Monoglyceriden 2 Gew.-% bis 40 Gew.-%, vorzugsweise 20 Gew.-% partialacetylierte Monoglyceride enthalten.

3. Wirkstoffhaltige, wasserfreie, lyophilisierte Emulsionszusammensetzung nach Ansprüchen 1 bis 2, dadurch gekennzeichnet, daß diese mindestens einen Wirkstoff aus der Wirkstoffgruppe der Prostaglandine, insbesondere PGE₁ enthalten.

4. Wirkstoffhaltige, wasserfreie, lyophilisierte Emulsionszusammensetzung nach Ansprüchen 1 bis 3, dadurch gekennzeichnet, daß sie als Gefrierschutzmittel/Gerüstbildner physiologisch verträgliche Mono-, Di- oder Oligosaccharide, insbesondere Laktose oder Zuckeralkohole wie Sorbit und/oder Mannit enthalten.

5. Wirkstoffhaltige, wasserfreie, lyophilisierte Emulsionszusammensetzung nach Ansprüchen 1 bis 4, dadurch gekennzeichnet, daß sie mindestens ein übliches Antioxidans, insbesondere aus der Gruppe der Tocopherole wie α-, β-, γ- oder δ-Tocopherol, vorzugsweise α-Tocopherol sowie deren physiologisch verträgliche Salze wie Phosphate, Succinate und Acetate und/oder physiologisch verträgliche Puffersalze enthält.

6. Wirkstoffhaltige, wasserfreie, lyophilisierte Emulsionszusammensetzung nach Ansprüchen 1 bis 5, dadurch gekennzeichnet, daß die innere, disperse Phase nach Rekonstitution mit Wasser mittlere Teilchendurchmesser von 0,1 µm bis 3 µm, vorzugsweise 0,2 µm bis ungefähr 1,0 µm aufweist.

7. Verfahren zur Herstellung einer wirkstoffhaltigen, wasserfreien, lyophilisierten Emulsionszusammensetzung gemäß Ansprüchen 1 bis 6, dadurch gekennzeichnet, daß in üblicher Weise eine wirkstoffhaltige Emulsion hergestellt wird, deren äußere, wässrige Phase anschließend mittels Gefriertrocknung entfernt wird.

## Claims

1. Active substance-containing anhydrous lyophilized emulsion composition, characterized in that the latter is consisting of at least one active substance, at least one cryoprotection agent and/or at least one bulking agent, glycerol polyethylene glycol ricinoleate or polyoxyethylene 660 12-hydroxystearate and diacetylated monoglycerides or a mixture of diacetylated and partially acetylated monoglycerides, in which glycerol polyethylene glycol ricinoleate resp. polyoxyethylene 660 12-hydroxystearate and diacetylated monoglycerides resp. the mixture of diacetylated and partially acetylated monoglycerides are present to each other in a ratio of 2:3 by weight, which, after reconstitution with water, has a particle size distribution corresponding to the original emulsion.

2. Active substance-containing anhydrous lyophilized emulsion composition according to Claim 1, characterized in that the mixture of diacetylated and partially acetylated monoglycerides contain 2 % by weight to 40 % by weight, preferably 20 % by weight, of partially acetylated monoglycerides.

3. Active substance-containing anhydrous lyophilized emulsion composition according to Claims 1 to 2, characterized in that the latter contains at least one active substance from the active substance group of prostaglandins, especially PGE₁.

4. Active substance-containing anhydrous lyophilized emulsion composition according to Claims 1 to 3, characterized in that it contains as cryoprotection agent/bulking agent physiologically tolerated mono-, di- or oligosaccharides, in particular lactose or sugar alcohols such as sorbitol and/or mannitol.

5. Active substance-containing anhydrous lyophilized emulsion composition according to Claims 1 to 4, characterized in that it contains at least one customary antioxidant, in particular from the group of tocopherols such as α-, β-, γ- or δ-tocopherol, preferably α-tocopherol, and the physiologically tolerated salts thereof, such as phosphates, succinates and acetates and/or physiologically tolerated buffer salts.

6. Active substance-containing anhydrous lyophilized emulsion composition according to Claims 1 to 5, characterized in that the internal disperse phase has, after reconstitu-tion with water, average particle diameters of 0.1 µm to 3 µm, preferably 0.2 µm to approximately 1.0 µm.

7. Process for the preparation of an active substance-containing anhydrous lyophilized emulsion composition according to Claims 1 to 6, characterized in that an active substance-containing emulsion is prepared in a conventional way and its external aqueous phase is subsequently removed by freeze-drying.

## Revendications

1. Composition d'émulsion lyophilisée exemple d'eau et contenant des substances actives, ***caractérisée en ce qu***'elle se compose d'au moins une substance active, d'au moins un agent antigel et/ou d'au moins un agent de lecture, de ricinoléate mixte de glycérol et de polyéthylèneglycol ou d'hydroxy-12 stéarate de polyéthylèneglycol-660, ainsi que de monoglycérides diacétylés ou d'un mélange de monoglycérides diacétylés et partiellement acétylés, le ricinoléate mixte de glycérol et de polyéthylèneglycol ou l'hydroxy-12 stéarate de polyéthylèneglycol-660 et les monoglycérides diacétylés ou le mélange de monoglycérides diacétylés et partiellement acétylés étant présents selon un rapport en poids de 2/3, et ***en ce qu***'après reconstitution avec de l'eau, elle présente une répartition granulométrique correspondant à celle de l'émulsion de départ.

2. Composition d'émulsion lyophilisée exemple d'eau et contenant des substances actives selon la Revendication 1, ***caractérisée en ce que*** le mélange de monoglycérides diacétylés et partiellement acétylés contient 2 % en poids à 40 % en poids, et de préférence 20 % en poids de monoglycérides partiellement acétylés.

3. Composition d'émulsion lyophilisée exemple d'eau et contenant des substances actives selon les Revendications 1 à 2, ***caractérisée en ce que*** celle-ci contient au moins une substance active du groupe des prostaglandines, en particulier PGE₁.

4. Composition d'émulsion lyophilisée exempte d'eau et contenant des substances actives selon les Revendications 1 à 3, ***caractérisée en ce que***, comme agent antigel/agent de structure, elle contient des mono-, di- ou oligosaccharides compatibles physiologiquement, en particulier du lactose ou des alcools de sucre comme le sorbitol et/ou le mannitol.

5. Composition d'émulsion lyophilisée exempte d'eau et contenant des substances actives selon les Revendications 1 à 4, ***caracterisée en ce qu***'elle contient au moins un antioxydant conventionnel, en particulier du groupe des tocophérols, tel que l'α-, le β-, le γ- ou le δ-tocophérol, et de préférence l'α-tocophérol, ainsi que leurs sels compatibles physiologiquement, tels que les phosphates, succinates et acétates, et/ou des sels tampons compatibles physiologiquement.

6. Composition d'émulsion lyophilisée exempte d'eau et contenant des substances actives selon les Revendications 1 à 5, ***caractérisée en ce que*** la phase intérieure dispersée présente, après reconstitution à l'eau, un diamètre moyen de particules de 0,1 µm à 3 µm, et de préférence de 0,2 µm à environ 1,0 µm.

7. Procédé de fabrication d'une composition d'émulsion lyophilisée exempte d'eau et contenant des substances actives selon les Revendications 1 à 6, ***caractérisé en ce qu***'on fabrique d'une manière conventionnelle une émulsion contenant des substances actives dont la phase extérieure aqueuse est ensuite éliminée par lyophilisation.
